Europäisches Patentamt

European Patent Office

Office européen des brevets

(19)

(11) Publication number: **0 309 429**

**A2**

(12)

# EUROPEAN PATENT APPLICATION

(21) Application number: **88850303.4**

(22) Date of filing: **14.09.88**

(51) Int. Cl.⁴: **C 12 Q 1/66**

(30) Priority: **23.09.87 SE 8703675**

(43) Date of publication of application:
**29.03.89 Bulletin 89/13**

(84) Designated Contracting States:
**AT BE CH DE ES FR GB IT LI NL SE**

(71) Applicant: **LIFE SCIENCE INTERNATIONAL AB**
**Box 2135**
**S-183 02 Täby (SE)**

(72) Inventor: **Lundin, Arne**
**Strandvägen 36**
**S-130 54 Dalarö (SE)**

**Lundin Karnell, Ulrika**
**Strandvägen 36**
**S-130 54 Dalarö (SE)**

(74) Representative: **Henningsson, Gunnar et al**
**Bergling & Sundbergh AB Box 7645**
**S-103 94 Stockholm (SE)**

(54) Luminometric assay of cellular ATP.

(57) A diagnostic means for luminometric assay of ATP, comprising a reagent carrier, optionally attached to a substrate, and a reagent for said assay in dried form on said carrier;

a diagnostic kit for luminometric assay of cellular ATP, comprising:

a) cuvettes containing buffering solution;

b) a first diagnostic means containing a bioluminescence reagent and a second diagnostic means for sampling, or, alternatively, a combined diagnostic means containing both reagent and sampling functions; and optionally

c) accompanying directions as how to use the kit;

a method for luminometric assay of cellular ATP in a liquid sample, comprising the steps:

a) sampling an aliquot of said sample with a sampling element that may contain pretreatment reagents, which during an incubation period affects the ATP level of the sample to make it a more reliable estimate of the cellular ATP level;

b) rapidly heating said aliquot without dilution to a controlled temperature around 100°C capable of almost instantaneous inactivation of ATP degrading enzymes present in said aliquot or in pretreatment reagents and furthermore capable of an almost complete release of cellular ATP but not so high as to destroy ATP;

c) transferring the aliquot from step b) above to a buffering solution together with a bioluminescence reagent; and

d) measuring light emission to determine the cellular ATP content.

Fig. 1A

Bundesdruckerei Berlin

EP 0 309 429 A2

**Description**

## Luminometric assay of cellular ATP

The present invention relates to new techniques for performing luminometric assay of cellular ATP (adenosine-5'-triphosphate) in a liquid sample, and the invention covers a diagnostic means and a diagnostic kit for such luminometric assay and also a method for determining cellular ATP in a liquid sample.

BACKGROUND

In clinical microbiology rapid quantitation of microbial cells in biological specimens, e.g. urine, blood sputum and wound exudates, would speed up the diagnosis of infectious diseases. A reliable diagnostic test performed while the patient is still with the doctor would allow an immediate onset of adequate therapy. Thus the test should not only be rapid but should also be simple enough to be performed as a "doctor's office test". Bacteriuria testing used in the diagnosis of urinary tract infection (UTI) is an example of a high volume test often performed as a doctor's office test (100-200 million tests per year world-wide). Presently available rapid methods for bacteriuria testing are, however, not completely reliable, i.e. they have either poor sensitivity (give false negative results) or poor specificity (give false positive results).

In veterinary medicine rapid determination of biomass should be possible to perform at the farm. A major problem in veterinary medicine is mastitis, i.e. inflammation of the cow udder, resulting increased numbers of lumphocytes in the milk. Mastitis testing can be performed at the farm by the California mastitis test (CMT). However, this test is influenced by personal judgement and gives only a semiquantitative estimate of the number of lymphocytes. The market for mastitis testing can be estimated to 5-10 million tests per year in Europe and USA.

In food industry the control of bacteriological quality is generally done with conventional culture techniques requi ring at least overnight incubation before results are available. This increases the storage time before e.g. fresh food can be delivered and may decrease the quality of the food. Furthermore storage space and costs are increased.

Biotechnical industrial processes are often extremely sensitive to infection by contaminating microorganisms. This is a problem in conventional biotechnical industries, e.g. breweries, but even more so in modern biotechnical industrial processes based on slow-growing mammalian cell cultures easily overgrown by infecting microorganisms or on advanced down-stream processing (purification etc.) highly susceptible to infecting microorganisms. Infection may very well become the factor that will limit scaling up of many otherwise very promising biotechnical processes from laboratory to industrial scale.

Although the present invention and its background is related to reagents and procedures used in luminescence analysis in general, the present disclosure will be illustrated with regard to the use of bioluminescence analysis such as those based on firefly luciferase reagents.

One of the most promising methods that has been advocated for rapid quantitation of cells in the situations given above is the luminometric quantitation of cellular ATP (A.Lundin, Analytical applications of bioluminescence: The firefly system, pp. 44-74, and M. Harber, Applications of luminescence in medical microbiology and hematology, pp. 189-219, in "Clinical and Biochemical Luminescence", Eds. L.J. Kricka and T.J.N. Carter, Marcel Dekker, Inc., New York, 1982). This method is based on the firefly luciferase reaction resulting in light emission proportional to ATP concentration in the sample. The light emission may be measured with luminometers using photomultipliers or diods as light detectors or with photographic techniques (e.g. Polaroid cameras).

Under physiological conditions the intracellular ATP concentration is similar in all cells and the amount of ATP per cell proportional to the intracellular volume. Firefly luciferase is almost completely specific for ATP and the linear range of the assay with commercially available reagents and instrumentation is $10^{-15}$ - $10^{-9}$ moles of ATP in an assay volume of 1 ml. Bacterial cells contain approximately $10^{-18}$ moles per cell and most mammalian cells contain approximately $10^{-16}$ moles per cell (A. Lundin, Analytical applications of bioluminescence: The firefly system, pp. 44-74, in "Clinical and Biochemical Luminescence", Eds. L.J. Kricka and T.J.N. Carter, Marcel Dekker, Inc., New York, 1982). Thus 1000 bacterial cells or 10 mammalian cells can be detected. In many situations this is an adequate sensitivity. In bacteriuria testing an often used cut-off limit between negative and positive samples is $10^5$ CFU/milliliter (CFU = colony forming units, i.e. one or several cells clumping together being counted as one colony on the culture plate). Bacteriuria testing by luminometric ATP assay has been shown to be the most reliable rapid bacteriuria test (H. Hallander, A. Kallner, A. Lundin and E. Österberg, Evaluation of rapid methods for the detection of bacteriuria (screening) in primary health care, Acta path.microbiol.immunol.scand. Sect. B, 94:39-49, 1986). This test also points to a possibility to differentiate between microbial and mammalian cells by a four step procedure:

    1) Specific lysis of mammalian cells with a detergent, e.g. tritonX-100.
    2) Degradation of all non-bacterial ATP by an ATP degrading enzyme system, e.g. apyrase.
    3) Extraction of bacterial ATP with simultaneous inactivation of ATP degrading enzymes.
    4) Determination of bacterial ATP by luminometric assay.

In e.g. mastitis testing mammalian cells can be specifically determined in the presence of microbial cells by using an extractant lysing mammalian but not microbial cells, e.g. tritonX-100. Mastitis testing by luminometric ATP assay has been described in the literature (T. Olsson, K. Sandstedt, O. Holberg and A. Thore, Biotechnology and Applied Biochemistry, 8, 361-9, 1986).

Several methods for the extraction of cellular ATP have been compared in various types of cells (A. Lundin and A. Thore, Comparison of methods for extraction of bacterial adenine nucleotides determined by firefly assay, Appl.Microbiol. 30:713-21; 1975, A. Lundin, Extraction and automatic luminometric assay of ATP, ADP and AMP, in "Analytical Applications of Bioluminescence and Chemiluminescence", Eds. L.J. Kricka, P.E. Stanley, G.H.G. Thorpe, pp. 491-501, Academic Press, London, 1984). From these studies it was concluded that the most reliable method was the extraction with trichloroacetic acid (TCA). A final concentration of 1-10% TCA in the extract is required. Algal, fungal and bacterial cells require higher TCA concentrations than mammalian cells.

Extraction with TCA (e.g. 5% in water) can be done by adding an equal volume of TCA to the sample directly in the assay cuvette. If the final assay volume is 1 milliliter, the volumes of sample and extractant shall not exceed 10 microliter or TCA will cause analytical interference with the firefly reaction. Thus in quantitation of bacterial cells the 1000 cells required for detection must be present in a 10 microliter volume, corresponding to $10^5$ cells per milliliter. Increased sensitivity can be obtained by adding more luciferase to the reagent or by using a more sensitive light detector. Each approach may be used to lower the detection limit at least 10 times, but the assay cost will increase making the method less attractive for routine purposes.

Alternative extraction methods involve various chemical or physical agents. Mammalian cells can often be extracted with lytic agents, e.g. detergents like tritonX-100, which do not cause analytical interference with the firefly reaction. In the presence of EDTA such extracts are stable, since ATP degrading enzymes require divalent metal ions for activity. Microbial or other cells with tough cell walls require more potent extractants like strong acids (e.g. trichloroacetic acid, perchloric acid), certain organic solvents (e.g. dimethylsulphoxide) or quarternary ammonium compounds (e.g. dodecyltrimethylammonium bromide).

Common features of all reliable extractants of bacterial ATP are that they rapidly penetrate cell walls and rapidly inactivate enzymes. Enzyme inactivation seems to be an inherent property of any reliable extractant of bacterial ATP. This is most likely due to a high intracellular concentration of ATP degrading enzymes and a slow release of the intracellular ATP through the cell wall during the extraction process. In methods requiring degradation of extracellular ATP a rapid inactivation of the ATP degrading enzyme system is also required. Thus in most analytical situations of practical importance the chemical extractant has to be enzyme inactivating. Since the luminometric determination of ATP is an enzymatic assay, it will be necessary either to dilute extracts extensively or to remove the enzyme inactivating extractant before the assay. TCA can be removed by extraction with diethyl ether and perchloric acid (another reliable extractant) can be removed by neutralisation and precipitation of the perchlorate anion using e.g. potassium hydroxide (A. Lundin and A. Thore, Comparison of methods for extraction of bacterial adenine nucleotides determined by firefly assay, Appl.Microbiol. 30:713-21, 1975). However, such procedures are time-consuming and not applicable to routine work in e.g. a doctor's office situation. Dilution of the extract may be acceptable in routine work but would decrease the sensitivity of the assay.

Among the extraction methods based on physical principles the only reliable method described for bacterial ATP is the rapid heating by a $\geqq 5$ fold dilution in boiling buffer (A. Lundin and A. Thore, Comparison of methods for extraction of bacterial adenine nucleotides determined by firefly assay, Appl.Microbiol. 30:713-21, 1975). The slight underestimation of bacterial ATP obtained with some bacteria would not be a major problem in routine applications. The extraction by dilution in boiling buffer also inactivates ATP degrading enzyme systems added for degradation of extracellular ATP. However, the boiling buffer method involves several practical problems, e.g. evaporation of water (resulting in an unknown concentration of the extracts) and the necessity to cool the extracts to room temperature before the assay. Thus extraction by dilution in boiling buffer is unsuitable for a doctor's office situation. Furthermore the dilution will give a decreased sensitivity.

An increased sensitivity can be obtained by concentrations of cells before the extraction. However, conventional concentrations methods, e.g. filtration or spinning down the cells in a centrifuge, easily disturbs the physiology of the cells resulting in decreased ATP levels (A. Lundin and A. Thore, Comparison of methods for extraction of bacterial adenine nucleotides determined by firefly assay, Appl.Microbiol. 30:713-21, 1975). The only method that seems safe from this point of view is equilibrium centrifugation in a density gradient (Ö. Molin, L. Nilsson and S. Ånséhn, Rapid detection of bacterial growth in blood cultures by bioluminescent assay of bacterial ATP, J.Clin.Microbiol. 18:521-5, 1983). However, neither this method nor any other described method for concentration of cells is suitable for a doctor's office situation.

Even in situations where the sensitivity of the assay is adequate, e.g. bacteriuria testing and mastitis testing, the method has not gained a widespread routine use. The major obstacle has been that the analytical procedure including pretreatment of samples is too complicated to meet the requirements in those situations where a rapid test result is of interst (e.g. doctor's office testing for bacteriuria or mastitis testing at the farm). As an example the simplest analytical procedure for bacteriuria testing that has been described (A. Lundin, H. Hallander, A. Kallner, U. Karnell Lundin and E. Österberg, Bacteriuria detection in an outpatient setting: Comparison of several methods including an improved assay of bacterial ATP, in "Rapid Methods and Automation in Microbiology and Immunology", Springer Verlag, Berlin, 1985, pp. 455-60) involves the following steps:

1) Mix 10 microliter urine and 10 microliter pretreatment reagent (0.2% tritonX-100 and 0.2% apyrase) in a cuvette.

2) Incubate at room temperature for 10 minutes.
3) Add 10 microliter 7.5% TCA for extraction of bacterial ATP and apyrase inactivation.
4) Add 1 milliliter firefly reagent in buffer.
5) Insert cuvette in instrument and measure light emission, $I_{sample}$.
6) Add 10 microliter ATP standard with a known concentration, $C_{standard}$, insert cuvette in instrument and measure light emission, $I_{sample+standard}$.
7) Calculate bacterial ATP concentration in urine, $C_{urine}$, according to the formula:

$$C_{urine} = \frac{I_{sample}}{I_{sample+standard} - I_{sample}} * C_{standard} \qquad (1)$$

With reagents resulting in a blank it is necessary to run a blank omitting the addition of urine in the procedure above and subtract the result calculated by the formula above from $C_{urine}$.

Although the procedure above is simple for a research laboratory or even for a routine clinical laboratory it is much too complicated for a doctor's office situation. It involves e.g. four 10 microliter pipettings and one 1 milliliter pipetting. In a doctor's office situation pipettings and especially microliter pipettings should be avoided. Thus even if the number of pipettings could be reduced by the use of standardized reagents the method would not be competitive with presently available stick methods. Manual pipettings might be avoided by automatic instrumentation, but the cost for such instruments would be too high to make the method competitive in a doctors office situation.

The present invention is based on four surpirising findings. The first finding is that it is possible to stabilize all reagents needed for biomass estimation by bioluminescence assay of ATP simply by drying them on one or several reagent carriers made of e.g. filter paper. This applies to pretreatment reagents as tritonX-100 and apyrase, to bioluminescence reagents as those based on firefly luciferase and finally to the ATP standard used for calibration of luminometric assays of ATP. Furthermore, it is possible to stabilize sample ATP on a sampling element made of e.g. filter paper by including EDTA or similar chelators of divalent metal ions in dried form on the sampling element and by drying the sample on the sampling element. The second finding is that a sampling element of fibrous material, e.g. filter paper, can be used for sampling a defined sample volume as well as for performing pretreatments as degradation of non-microbial ATP or extraction of cellular ATP. The third finding is that it is possible to perform heat extraction of cellular ATP with simultaneous inactivation of ATP degrading enzyme systems on the sampling element and without the dilution inherent in the boiling buffer procedure. The fourth finding is that neither sample ATP nor bioluminescence reagent will be permanently bound to the fibrous material but can be dissolved in assay buffer rapidly enough for a convenient assay procedure. These findings will be illustrated in the following description of the invention.

The principl object of the present invention is to provide new rapid techniques for facile determination of bio-mass, i.e. quantitation of microbial or other types of living cells, whereby the practical problems involved in diagnosis or quality control are eliminated or at least significantly reduced.

For this and other objects which will be clear from the following description the present invention provides for a diagnostic means, such as a stick, for luminometric assay of cellular ATP, which stick comprises a substrate and attached thereto a reagent carrier on which a reagent in dried form to be used for luminescence analysis is arranged. Instead of a stick the reagent carrier can be enclosed in a container, e.g. a tube, so that it can easily be transferred to the buffer in which the luminometric assay is to be performed or so that the buffer can be added to the container and the assay performed in this container.

The term "diagnostic" as used herein is to be broadly interpreted and is intended to cover both regular diagnosis and other aspects, such as quality control of a certain product.

In a preferred embodiment of the invention said reagent carrier is constituted by a fibrous pad. A particularly preferred bioluminescence reagent is based on firefly luciferase.

It is preferred that the diagnostic stick as referred to above also comprises a sampling element attached to said substrate or that one substrate contains the bioluminescence reagent carrier and another substrate the sampling element. In the former situation the sampling element need not always be present as a separate element but may in some analytical situations be inherent in the reagent carrier.

The sampling element is preferably constituted by a fibrous pad.

In accordance with the present invention it has been surprisingly found that firefly luciferase reagent can be stabilized in dried form on a reagent carrier, such as a pad made of porous material, e.g. filter paper. This arrangement has been found to result in an astonishing high stability, and firefly reagent pads according to the invention made for example of filter paper can be stored at room temperature for several months. Furthermore, the bioluminescence reagent is easily redissolved in buffer solution. Such a bioluminescence reagent pad can be conveniently handled without risk for contamination if attached to a stick or enclosed in a container. In the following description the reagent pad is assumed to be attached to a diagnostic stick but using a reagent pad enclosed in a container would give a similar analytical procedure.

A diagnostic stick according to the invention including a sampling element is convenient to use in actual practice, and a defined sample volume can be obtained by soaking the sampling element in the sample subject to assay for a few seconds. With an appropriate sampling element material, e.g. filter paper, the accuracy of the sample volume is generally within 10 percent, which is acceptable in most analytical situations. Furthermore, according to the invention it has been surprisingly found that pretreatment reagents, such as tritonX-100 and apyrase, can be stabilized in dried form in the sampling element. In a special analytical situation where ATP can be extracted with an extractant not interfering with the firefly reaction it is possible to have the extractant present in dried form on or in the sampling element. One such situation is mastitis testing performing the extraction of lymphocytes in milk using a sampling element consisting of filter paper with tritonX-100 and EDTA is dried form. Furthermore, according to the present invention it has been surprisingly found that the sample ATP in this and similar situations can be stabilized by including EDTA or similar chelators of divalent metal ions and/or by rapidly drying the sample. Thus the sample can be sent to a central laboratory performing the assay of ATP.

According to another aspect of the invention there is provided a diagnostic kit for luminometric assay of cellular ATP, said kit comprising in combination:

    1) cuvettes containing buffering solution;

    2) a first stick containing a bioluminescence reagent and a second stick for sampling, or, alternatively, a combined stick containing both reagent and sampling functions; and optionally,

    3) accompanying directions as how to use the kit.

As a supplement such a diagnostic kit can further comprise a calibration stick containing a known amount of ATP standard stabilized in dried form.

According to yet another aspect of the invention there is provided a method for luminometric assay of cellular ATP in a liquid sample, said method comprising the steps:

    1) sampling an aliquot of said sample;

    2) optional pretreatment of said aliquot e.g. to degrade non-microbial ATP;

    3) treating said aliquot without dilution by rapid heating to a temperature capable of inactivating ATP degrading enzyme systems present in said aliquot or in pretreatment reagents but not to a temperature so high as to destroy ATP, and maintaining said temperature for a period of time adequate to completely inactivate said enzyme systems and to release ATP from the cells;

    4) transferring the aliquot from step c) above to a buffering solution together with a luminescent reagent; and

    5) measuring light emission to determine the ATP content.

In such a method it is preferred that the heat treatment under step c) is constituted by microwave heating or steam heating.

A basic concept of the present invention is to provide for heat extraction without the dilution inherent in the boiling buffer method. The requirements on a heat extraction without dilution are:

    1) The temperature has to rise almost instantaneously (within a few seconds) to at least 70°C and preferably to 100°C to avoid degradation of ATP by enzyme systems present in the cells or in pretreatment reagents.

    2) The temperature should be maintained at this elevated temperature during the time needed for complete inactivation of ATP degrading ensyme systems and release of cellular ATP (a few seconds to a few minutes depending on type of cell and the medium in which they are suspended), but the temperature may never during this time become so high that ATP is destroyed.

    3) The heat has to be transferred without contact with the heating device to avoid carry over from one sample to the other.

The preferred temperature for heat extraction without dilution is around the boiling point of water (90-110°C). Sufficient heat without dilution or carry over problems can be obtained e.g. by infrared radiation or by passing an electric current through the sampling element in in-built conductors. With these sources of heat overheating resulting in degradation of ATP can only be avoided by relatively complicated methods requiring detailed optimization of heating devices and analytical procedures. However, there are at least two very simple ways to rapidly transfer heat avoiding dilution, overheating and carry over problems. One way is to use microwaves, which inherently only transfer heat to the sample as long as the water has not evaporated and thus keeps the temperature close to 100°C. With microwaves there is also no risk of inactivating the bioluminescence reagent if this does not contain water.

Another way to obtain rapid heat extraction without dilution, carry over or overheating problems is to use steam generated by boiling water in an open vessel. A constant flow of steam with a temperature of 100°C is very simple to achieve with low cost components. In such a flow the temperature of the sampling element is rapidly raised to close to 100°C and there is no risk of overheating. The extraction time can even be predetermined by injecting a limited amount of water into the boiling vessel. The boiling vessel can be conveniently heated by electricity. A small deposit of water from the steam on the sampling element may be obtained but will not seriously influence the assay result.

According to the principles described above rapid heat extraction without dilution, overheating or carry over problems can be obtained with the present invention. This solves the problem involved in the use of chemical extractants for releasing ATP from bacteria and other microorganisms, which extractants usually cause analytical interference disturbing the assay. Furthermore, the dilution inherent in the boiling buffer method and the time needed for cooling the extract to room temperature is avoided.

As indicated above examples of heat sources for the rapid heating are microwave radiation and steam heating.

In using microwave heating the diagnostic stick with the sampling element is inserted into a small microwave oven for a few seconds. Such microwave ovens can be produced at reasonable prices and need not have physical dimensions larger than a few liters. A major advantage of microwave heating is that it is instantaneous and will by its inherent properties cease when the water in the sample has evaporated. Over heating resulting in degradation of ATP will thus not appear. With a diagnostic stick containing both sampling element and reagent carrier there is no risk that the bioluminescence reagent part of the stick will be heated, since said part does not contain water except in the special embodiment of the invention where the sampling element is inherent in the reagent carrier (an embodiment that cannot be combined with heat extraction). Furthermore it is generally possible to physically separate the sampling element and the reagent carrier so that only the sampling element is exposed to the microwaves.

Steam heating is another preferred technique for providing rapid heating to an enzyme inactivating temperature of the sample. Steam heating can be provided by injecting water into an electrically heated chamber with a hole through which the steam passes. The part of the diagnostic stick to be heated is positioned in front of this hole. In using a combined stick the bioluminescence reagent must, of course, not be heated. In this case it may thus be more practical to use a diagnostic stick wherein the bioluminescence reagent is well separated from the sampling part of the stick, or even two separate sticks rather than a combined stick. The use of steam heating is advantageous in that it is based on very low cost components and may accordingly be the most competitive technique for many applications.

Detailed illustration of the invention

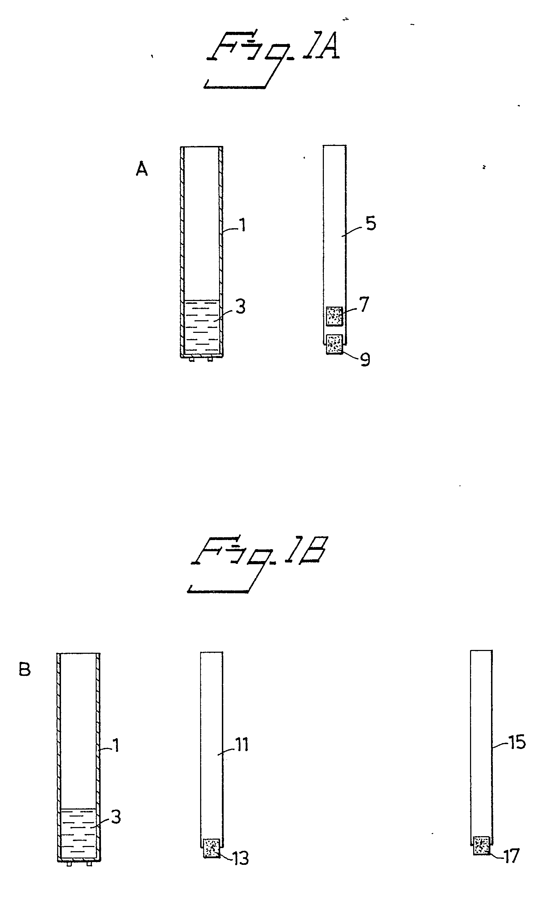

In the following the invention will be further illustrated by examples with reference to the appended drawing, wherein Fig. 1a illustrates an embodiment with a combined diagnostic stick, and Fig. 1b illustrates an embodiment using separate sampling and firefly reagent sticks.

In Fig. 1a there is shown a cuvette generally designated 1 containing a buffer solution 3. Furthermore there is illustrated in Fig. 1a a combined diagnostic stick generally designated 5 including a firefly reagent pad 7 and a sampling pad 9 arranged separately on stick 5.

Fig. 1b shows a corresponding cuvette 1 containing buffer solution 3. Furthermore Fig. 1b illustrates separate sampling and firefly reagent sticks, the sampling stick 11 containing at one end thereof a sampling pad 13, and the reagent stick 15 containing at one end thereof a firefly reagent pad 17.

Example 1 (Kit and analytical procedure for estimating bacterial cells in absence of other types of cells)

The components of the kit are as follows:

1) Cuvettes (LKB-Wallac, Turku, Finland) containing buffer (0.1 M tris-acetate, pH 7.75, containing 2 mM EDTA).

2) Sticks with sampling and firefly reagent pads of filter paper (Schleicher & Schüll nr 2668/2) according to Fig. 1a. In the absence of other types of cells the amount of bacterial cells may be estimated without pretreatment reagents in the sampling pad, since extracellular ATP generally is very low in this situation.

3) Calibration stick with a known amount of dried ATP standard on a special pad called "calibration pad". The amount of ATP will correspond to a known concentration of cells in the sample. The calibration stick is used for calibrating the instrument so that the result can be converted to number of cells per milliliter.

The analytical procedure is as follows:

1) Soak the combined stick in sample for a few seconds to allow sampling pad to become completely wetted (coefficient of variation of sample volume with this procedure using suitable filter paper pads is less than 10%).

2) Apply heat to the sampling pad in a microwave oven with a frequency of 2450 MHz and an applicator for small volume samples so designed as to obtain close to 100° C within a few seconds and to maintain this temperature as long as there is water left in the sampling pad and at least 10 seconds.

3) Put the combined stick into ready made cuvette containing 1 milliliter buffer and allow sample ATP as well as firefly reagent to be dissolved in the buffer (takes less than a minute if the content of the cuvette is occasionally mixed using an efficient mixer).

4) Insert cuvette in instrument and measure light emission, $I_{sample}$ (proportional to cellular ATP level, which is proportional to amount of cells).

5) Put calibration stick into the cuvette and mix as in step 3.

6) Insert cuvette in instrument and measure light emission, $I_{sample+standard}$.

7) Calculate sample ATP concentration, $C_{sample}$, according to the formula:

$$C_{sample} = \frac{I_{sample}}{I_{sample+standard} - I_{sample}} *C_{standard} \qquad (2)$$

The value of $C_{standard}$ is easily calculated by assuming that the known amount of ATP standard applied on the calibration pad is in the same volume as soaked up by the sampling pad. This volume can be estimated by weighing a few sampling pads before and after sampling.

With reagents resulting in a blank it is necessary to run a blank by the same procedure as above but using e.g. distilled water instead of sample. The blank result is then subtracted from the uncorrected sample result. The procedure above is rather complicated but would be generally applicable even in situations with a sample containing substances strongly inhibitory to the firefly reaction. If there is no such inhibition the calibration procedure (steps 5 and 6) can be omitted on all but one sample in each series of assays.

The above procedure can also be used if extracellular ATP is not negligible provided that an ATP degrading enzyme system, e.g. apyrase, is applied in dried form to the sampling pad. In this situation an incubation step (10 minutes at room temperature) should be included between steps 1 and 2.

Example 2 (Kit and analytical procedure for bacteriuria testing)

A bacteriuria kit prototype was developed and consisted of the following components:
1) A Luminometer 1250 (LKB-Wallac, Turku, Finland) and a steam extraction device. The latter was a household egg boiler with a tube on top to guide the steam and an arrangement for holding the sampling stick.
2) Sampling and calibration sticks were made from plastic bacteriological loops (a 1 microliter volume O-loop with a long handle). A fracturing point was applied by a special pair of nippers approximately 2 cm above the end of the loop, i.e. the O. The O was cut by a special pair of cutting nippers so that a pad of filter paper (Schleicher & Schüll No. 2668/2, $\varnothing$ approx. 5 mm) could be squeezed in. With the sampling stick the pad was soaked in a solution of 0.1% tritonX-100 and 0.1% apyrase (Sigma A7646) in distilled water. With the calibration stick the pad was soaked in 10$\mu$m ATP Standard (LKB-Wallac, Turku, Finland). Firefly reagent pads were prepared by applying 20 microliters of a solution in 1000 microliters of water of one vial of ATP Monitoring Reagent (LKB-Wallac, Turku, Finland). All types of pads were dried over night in the cold room (4-6°C) under vacuum. Sampling and calibration sticks were stored in plastic bags and firefly reagent pads in cuvettes with lids (LKB-Wallac, Turku, Finland).
3) Plastic Ellerman tubes with lids containing 1 milliliter of 0.1 M Tris-acetate buffer, pH 7.75 containing 2 mM EDTA.
The analytical procedure was as follows:
1) Soak sampling pad in urine until completely wet (takes a few seconds).
2) Incubate sampling stick at room temperature for 10 minutes.
3) Pour buffer into cuvette with firefly reagent pad.
4) Expose sampling pad to steam flow from steam extraction device for 10 seconds.
5) Put sampling stick in cuvette containing firefly reagent pad and break handle at fracturing point. Mix so that contents of both pads are dissolved in buffer (takes 1 minute).
6) Insert cuvette in luminometer and measure light emission, $I_{sample}$.
7) Put calibration stick with pad containing a known amount of ATP in cuvette, break handle at fracture point and dissolve ATP (takes 1 minute) and measure light emission, $I_{sample+standard}$.
8). Calculate ATP concentration in urine using equation (1).
The calibration step may be avoided in a routine procedure with carefully standardized instrumentation and reagents, since the ATP Monitoring Reagent is extremely stable when dried on pads. In a commerical kit a combined stick may be used.

Example 3 (Kit and analytical procedure for mastitis testing)

A mastitis testing prototype kit was developed and consisted of the following components:
1) A milk sampler consisting of a stick with a filter paper (Schleicher & Schüll No. 2668/2 ($\varnothing$ approx. 5 mm)) attached to a plastic stick with a fracturing point. The filter paper had been prepared by applying 20 microliters of a solution containing 0.6% tritonX-100 and 250 mM of EDTA (Merck, Darmstadt, FRG) followed by drying over night at room temperature. In a special version of the kit the plastic stick was contained in a plastic container excluding humidity and containing moisture absorbing material (silica gel).
2) Cuvettes with lids (LKB-Wallac, Turku, Finland) with firefly reagent pad identical to the one described above for bacteriuria testing.

3) Plastic Ellerman tubes with lids containing 1 milliliter of 0.1 M Tris-acetate buffer, pH 7.75, containing 2 mM EDTA.

4) Calibration sticks similar to those described in Example 1 containing a known amount of ATP but also tritonX-100 and EDTA as with the milk sampler. Firefly reagent sticks similar to those described in Example 1 but without sampling pad.

5) A modified Polaroid camera (home-built) allowing light emission from inserted cuvettes to be measured. The light intensity could be estimated from the diameter of the light area on the film. Alternatively a series of various grey filters inserted between the cuvette and the film could be used to obtain a pattern of light areas from which the light intensity could be estimated.

The analytical procedure when performed at the farm was as follows:

1) Allow a jet of milk to soak the filter paper of the milk sampler stick.

2) Pour buffer into cuvette containing firefly reagent pad.

3) Put milk sampler stick in cuvette, break handle at fracturing point and mix occasionally during 1 minute.

4) Position sample in Polaroid camera together with a cuvette prepared according to steps 2 and 3 but with a calibration stick instead of milk sampler stick. Allow light from firefly reaction to expose Polaroid film, develop film and read result as indicated above.

The ATP assay can also be performed in the laboratory using conventional instrumentation, e.g. automatic luminometers as Luminometer 1251 (LKB-Wallac, Turku, Finland). In this case the special version of the kit including the plastic container should be used. After applying the milk the plastic container is sealed and transported to the laboratory. During the transport ATP is stabile due to the presence of EDTA in the sampling pad and the drying effect of the silica gel. In the laboratory the milk sampler is put in a cuvette with buffer and the automatic luminometer performs the additions of firefly reagent and ATP standard. The automatic luminometer can be connected to a computer for rapid calculation of results.

## Claims

1. A diagnostic means for luminometric assay of ATP, comprising a reagent carrier, optionally attached to a substrate, and a reagent for said assay in dried form on said carrier.

2. A diagnostic means according to claim 1, wherein said carrier is constituted by a fibrous pad.

3. A diagnostic means according to claim 1 or 2, wherein said reagent is a bioluminescence reagent, such as one based on firefly luciferase.

4. A diagnostic means according to claim 1 or 2 comprising said reagent carrier attached to a substrate.

5. A diagnostic means according to claim 4, wherein a sampling function is inherent in the reagent carrier.

6. A diagnostic means according to claim 4 or 5, wherein the sampling function is obtained by a fibrous pad.

7. A diagnostic kit for luminometric assay of cellular ATP, comprising:

a) cuvettes containing buffering solution;

b) a first diagnostic means containing a bioluminescence reagent and a second diagnostic means for sampling, or alternatively, a combined diagnostic means containing both reagent and sampling functions; and optionally,

c) accompanying directions as how to use the kit.

8. A diagnostic kit according to claim 7 further comprising a diagnostic means containing a reagent carrier with a known amount of ATP standard in dried form.

9. A method for luminometric assay of cellular ATP in a liquid sample, comprising the steps:

a) sampling an aliquot of said sample with a sampling element that may contain pretreatment reagents, which during an incubation period affects the ATP level of the sample to make it a more reliable estimate of the cellular ATP level;

b) rapidly heating said aliquot without dilution to a controlled temperature capable of almost instantaneous inactivation of ATP degrading enzymes present in said aliquot or in pretreatment reagents and furthermore capable of an almost complete release of cellular ATP but not so high as to destroy ATP;

c) transferring the aliquot from step b) above to a buffering solution together with a bioluminescence reagent; and

d) measuring light emission to determine the cellular ATP content.

10. A method according to claim 9, wherein the heat extraction under step b) is constituted by microwave heating or steam heating.

11. A method according to claim 9 or 10, wherein said controlled temperature is from about 90°C to about 110°C.

Fig. 1A

Fig. 1B